(19) Europäisches Patentamt / European Patent Office / Office européen des brevets

(11) **EP 1 824 521 B1**

(12) **EUROPEAN PATENT SPECIFICATION**

(45) Date of publication and mention
of the grant of the patent:
**07.01.2015 Bulletin 2015/02**

(51) Int Cl.:
*A61K 49/00* *(2006.01)*    *A61K 47/48* *(2006.01)*

(21) Application number: **05804156.7**

(22) Date of filing: **15.11.2005**

(86) International application number:
**PCT/IB2005/053763**

(87) International publication number:
**WO 2006/054240 (26.05.2006 Gazette 2006/21)**

(54) **ULTRASOUND CONTRAST AGENTS FOR MOLECULAR IMAGING**

ULTRASCHALLKONTRASTMITTEL FÜR MOLEKULARE BILDGEBUNG

AGENTS DE CONTRASTE ULTRASONORES POUR IMAGERIE MOLECULAIRE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **19.11.2004 EP 04105948**

(43) Date of publication of application:
**29.08.2007 Bulletin 2007/35**

(73) Proprietor: **Koninklijke Philips N.V.
5656 AE Eindhoven (NL)**

(72) Inventors:
• **WILLARD, Nicolaas, P.**
**NL-5656 AA Eindhoven (NL)**
• **VAN BOMMEL, Ties**
**NL-5656 AA Eindhoven (NL)**

(74) Representative: **Verweij, Petronella Danielle et al
Philips Intellectual Property & Standards
P.O. Box 220
5600 AE Eindhoven (NL)**

(56) References cited:
**WO-A-2004/068405    WO-A-2005/070473
US-A1- 2003 003 054**

• **WEITSCHIES W ET AL: "PARTIKULAERE
KONTRASTMITTEL ZUR INTRAVASALEN
APPLIKATION" PHARMAZEUTISCHE ZEITUNG,
GOVI VERLAG, ESCHBORN, DE, vol. 140, no. 38,
21 September 1995 (1995-09-21), pages 9-16,
XP000990911 ISSN: 0031-7136**
• **BEKEREDJIAN R ET AL: "Potential of gold-bound
microtubules as a new ultrasound contrast
agent" ULTRASOUND IN MEDICINE AND
BIOLOGY, NEW YORK, NY, US, vol. 28, no. 5, 1
May 2002 (2002-05-01), pages 691-695,
XP004366389 ISSN: 0301-5629 cited in the
application**

**Description**

The invention relates to a new type of ultrasound contrast agent (UCA) for molecular imaging as well as the method of imaging therewith.

In the last 15 years a number of safe and practical ultrasound contrast agents (UCAs) have been developed, such as gas-filled microbubbles, which enhance Doppler signals, and shell encapsulated droplets. (Hall C.S. et al. (2000) J. Acoust. Soc. Am. 108 (6), 3049-3057).

Ideally an ultrasound contrast agent should have as many as possible of the following features:

- Stable and sufficient lifetime in blood, e.g. allowing a detection in the targeted organ during 30 minutes or more;
- A particle size of less than 8 micron, so as to enable them to pass through blood capillaries;
- Non toxic, or acceptable toxicity;
- Sufficient reflection enhancement;
- Ease of production and clinical use;
- Allowing highly specific targeting.

Moreover, the ultrasound contrast agent should preferably be applicable with the existing ultrasound imaging systems, such as the Philips Ultrasound Imaging System.

Different particles comprising metals or metal oxides with magnetic properties have been developed for use as contrast agents for magnetic resonance imaging (MRI). US 2002/0136693 describes agents for diagnostic purposes, which contain magnetic particles comprising a magnetic double metal oxide/hydroxide or a magnetic metal and optionally a complexing agent. US 2003/0082237 describes nanoparticles, which are structured into spheres having an inner and outer layer of vesicles by block copolypeptides or homoplymer polyelectrolytes. Either the outer or inner layer of nanoparticles can comprise metals or metal oxides, which are optionally functionalized for site-selective medical imaging.

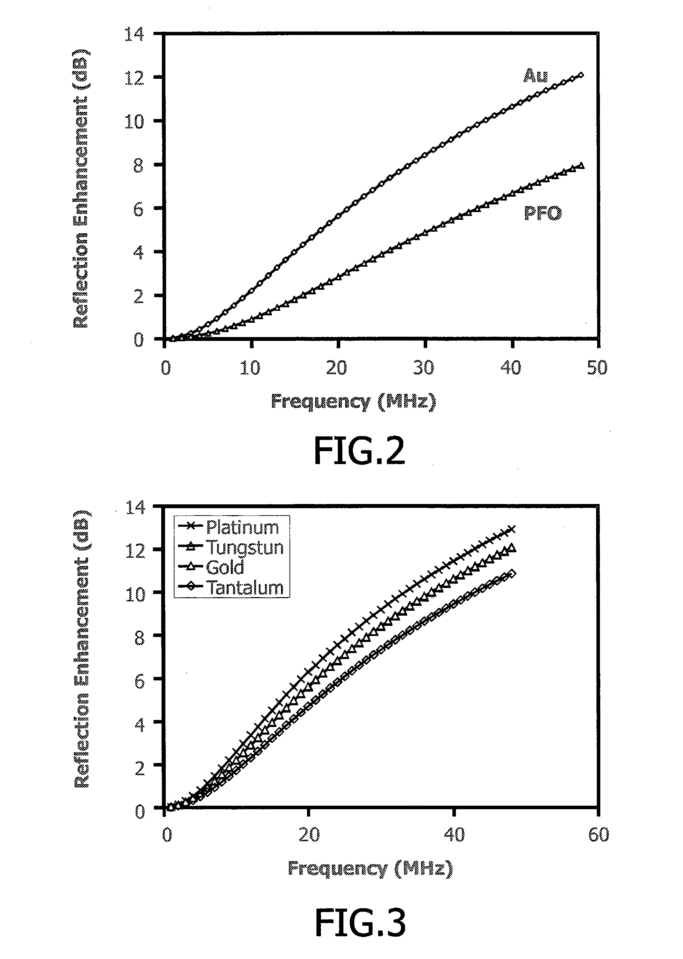

Metal nanoparticles can be used as ultrasound contrast agent. Especially targeted metal nanoparticles are of interest due to their tissue specific property resulting in higher local ultrasound contrast agent concentration, thus an increased reflection enhancement, and thus having the property of obtaining molecular information with these stable agents. Non-targeted metal nanoparticle ultrasound contrast agents will not accumulate at the required tissue and their concentration will not be high enough to be detectable at lower frequencies. By using higher frequencies a significant increase in reflection enhancement can be obtained as depicted in figure 2, 3 and 4. Nevertheless, high frequencies cannot be used up to now for e.g. organ studies, because of the penetration depth limitation as shown in table 1.

Table 1 Frequency, resolution and penetration of ultrasound.

| Frequency | Resolution | Penetration |
|-----------|------------|-------------|
| 7,5 MHz | 210 $\mu$m | 50-70 mm |
| 10 MHz | 158 $\mu$m | 35 mm |
| 22 MHz | 72 $\mu$m | 8 mm |
| 30 MHz | 52 $\mu$m | 4 mm |
| 50 MHz | 31 $\mu$m | 2 mm |
| 75 MHz | 21 $\mu$m | 1,5 mm |

Note that this table reflects a typical example of resolution and penetration dependency of the frequency for a given ultrasound equipment. The resolution and penetration are dependent upon the frequency of the transducer. But two transducers of the same frequency do not always have the same resolution and penetration.

Increasing the concentration of metal nanoparticles would enhance ultrasound reflectivity, notably at relatively low frequencies for which ultrasound radiation has an adequate penetration depth. Patent WO 02/11771 and Bekeredjian et al. (2002), Ultrasound Med. & Biol. 28(5), 691-695) describe the potential use of gold-bound microtubules as an ultrasound contrast agent. Such gold-bound microtubules displayed longer persistence of contrast activity than conventional contrast agents (microbubbles). However, absolute intensities were generally lower. There is thus a need for alternative compounds comprising metal nanoparticles, which lead to locally high concentrations of metal nanoparticles. High-contrast optoacoustic imaging using metallic nanoparticles with a dimension in the range of 1 to about 3000nm are known from the international applicaton WO2004/068405. Gadolinium particles encapsulated in microsphere shells for asue as a contrast agent are known from the US-patent application US2003/0003054.

An object of the present invention is to provide alternative ultrasound contrast agents (UCA) for molecular imaging as well as a method of imaging therewith. An advantage of the present invention is the provision of compounds which lead to locally high concentrations of metal nanoparticles.

In one aspect the present invention as defined in Claim 1 relates to a contrast agent for medical diagnostics and imaging which comprises a plurality of metal nanoparticles wherein said plurality of metal nanoparticles are encapsulated in a non-proteinaceous biocompatible or biodegradable matrix particle and/or attached to a non-proteinaceous biocompatible or biodegradable matrix particle, the matrix of the matrix particles being selected from the group consisting of a carbohydrate, a lipid, a synthetic polymer, an aqueous liquid, a surfactant and an organic liquid, or a mixture thereof. Such a matrix can be for example the shell of a vesicle. The metal nanoparticles can have, according to certain embodiments, an acoustic impedance of at least $35.10^5$ g/cm$^2$s or above $50.10^5$ g/cm$^2$s. The metal nanoparticles can have, according to certain embodiments, a diameter between 1 and 100 nm, or between 1 and 50 nm. The metal of said metal nanoparticles can be , according to certain embodiments, a non-magnetic metal such as gold, silver, platinum, palladium, tungsten or tantalum, rhenium, or a mixture thereof. The metal of said metal nanoparticles can be, according to certain embodiments a noble metal. The matrix particles of the contrast agents can have a diameter between 1 nanometer and 10 micrometer, e.g. between 1 and 8 micrometer or between 25 and 250 nanometer depending upon specific applications. The matrix particles comprising at least 2%, at least 5%, at least 10%, at least 20 % or even at least 50 % (vol/vol) of metal nanoparticles.

According to certain embodiments said metal nanoparticles are present in a concentration of at least 5% (volume/volume) in the aforementioned matrix. According to certain embodiments one or more targeting molecules can be attached to said matrix particle and/or to the surface of the metal nanoparticles.

The invention further relates to the use as defined in Claim 13 of a non-proteinaceous matrix particles comprising a plurality of metal nanoparticles for the manufacture of an ultrasound contrast agent, wherein said matrix is being selected from the group consisting of a carbohydrate, a lipid, a synthetic polymer, an aqueous liquid and an organic liquid, or a mixture thereof.

The invention may be applied in a method of gaining information about an animal or human patient, e.g. imaging or of diagnosis, the animal or human patient having been administered a contrast agent of the present invention, the method comprising: performing an ultrasound imaging examination of the animal or human.

The invention may be applied in a method of imaging an isolated tissue sample of organ, which method comprises administrating the contrast agent of the present invention to said tissue sample or organ and performing an ultrasound imaging examination thereof.

In one aspect the invention relates to a contrast agent for medical imaging or diagnostics comprising a plurality of metal nanoparticles characterized in that the plurality of metal nanoparticles are encapsulated in a non-proteinaceous biocompatible or biodegradable matrix particle. Alternatively, or in addition, the plurality of metal nanoparticles are attached to a non-protein biocompatible or biodegradable matrix particle. The matrix material of these particles can be any selected from the group consisting of a carbohydrate, a lipid, a synthetic polymer, an aqueous liquid, a surfactant and an organic liquid, or a mixture thereof. In one embodiment, the matrix of the contrast agent is the shell of the vesicle.

Another aspect relates to the use of a non-protein matrix particle comprising a plurality of metal nanoparticles for the manufacture of an ultrasound contrast agent, wherein the matrix of the particles is selected from the group consisting of a carbohydrate, a lipid, a synthetic polymer, an aqueous liquid and an organic liquid, a surface active compound, or a mixture thereof.

Another aspect of the invention may be applied in a method of imaging or diagnosis of a human or animal patient to whom a contrast agent comprising the above mentioned non-proteinaceous matrix particles have been administered, the method comprising performing an ultrasound imaging examination of the animal or human. The method may include administration of a contrast agent comprising the above mentioned non-proteinaceous matrix particles to the animal or human patient.

The contrast agent of the present invention can comprise a plurality of metal nanoparticles within one matrix component or can comprise in its matrix a plurality of metal nanoparticles. According to an embodiment of the present invention, the presence of metal particles in a matrix allows the use of metals which have a less preferred metal ion leaching or which could have toxic effects.

The matrix particles according to the present invention can be furthermore targeted using specific target agents such as cell, cell membrane, cell wall or body, e.g. Golgi body, tissue, microorganism, e.g. parasite, or biomolecule, e.g. protein, DNA or RNA specific target agents, of which antibodies or fragments thereof are only one example.

The plurality of metal nanoparticles associated with a matrix particle is an acoustic reflector due to the strong acoustic impedance difference with body tissue and has the advantage over current commercial UCAs, e.g. microbubbles of being stable and can be modified in the same way as current targeted contrast agents.

With the clustered metal nanoparticles of the present invention sufficient reflection enhancement can be obtained at the frequencies of e.g. 1 to 20 MHz, which are normally used for ultrasonic imaging of organs or tissue.

The matrix particles of the present invention can furthermore be used for drug delivery by coating the matrix with a therapeutic agent or by encapsulating the therapeutic agent in the matrix.

Particular embodiments of the present invention relate to contrast agents comprising a plurality of metal nanoparticles comprising metal particles having preferably an acoustic impedance above $35.10^5$ g/cm$^2$s or even more

preferably, having an acoustic impedance above $50.10^5$ g/cm$^2$s. Particular embodiments of the present invention relate to contrast agents comprising a plurality of metal nanoparticles wherein the metal is a non-magnetic metal. Examples hereof are gold, silver, platinum, palladium, tungsten or tantalum, rhenium, or a mixture thereof. According to a further embodiment the metal particles in the matrix particles comprise a metal which is a noble metal or a mixture of one or more noble metals with other metals, e.g. gold, silver, platinum, palladium, tungsten or tantalum, rhenium. According to a more particular embodiment of the invention, the metal nanoparticles in the matrix particle are made of gold.

Preferably, the metals being used are good acoustic reflectors (i.e. having a high acoustic impedance) and are noble metals. Gold and platinum have these two features.

Optionally, the metal particles comprise a metal oxide or have a stable thin oxide layer.

Another aspect of the invention relates to the use of the matrix particles comprising a plurality of metal nanoparticles of the invention as an imaging or a diagnostic agent, more particularly as an ultrasound contrast agent in ultrasound imaging, e.g. targeted ultrasound contrast imaging. Thus, the invention relates to the use of the matrix particles comprising a plurality of metal nanoparticles having one or more of the above-described characteristics in the production of a contrast agent, for use in ultrasound contrast imaging. This includes the use of the matrix particles comprising a plurality of metal nanoparticles for the visualization of tissue or parts thereof, as well as their use in the detection of specific targets such as, but not limited to, cellular markers, pathogens, etc.

Moreover, according to a particular aspect of the present invention, the matrix particles comprising a plurality of metal nanoparticles can also be detected using other imaging means allowing the use of the particles of the invention for combined imaging techniques.

Another aspect of the present invention is a method of imaging or diagnosis comprising administration of a contrast agent according to the present invention to an animal or human patient, and performing an ultrasound imaging examination of the animal or human. Alternatively, according to another aspect of the invention the contrast agent is administered to an animal or human tissue for diagnosis ex vivo.

The present invention relates to the use of matrix particles comprising a plurality of metal nanoparticles in ultrasound contrast agents as well as to the preparation and design of ultrasound contrast agents.

The matrix particles for use as contrast agents in the bloodstream preferably have a diameter of less than 10 micrometer, e.g. 8 micrometer, particularly about 3 micrometer or down to about 1 micrometer. Contrast agents which should have the ability to penetrate through the walls of the blood vessels preferably have a diameter in the nanometer range, e.g. the present invention provides matrix particles with a diameter between 250 nm and 1nm, and more particularly between 100 and 25 nm. Matrix particles with a diameter below 25 nm will have a short retention time in the body and are suitable for applications where short retention time is important. In one aspect of the present invention the particles preferably have enough body retention time, allowing targeting of the ultrasound contrast agent and/or performing the ultrasound examination of the patient, before they are degraded and/or excreted by the body. The metal nanoparticles in the matrix particle according to the present invention have a diameter of between 1 to 100 nm, preferably less than 50 nm, more particularly 30 nm or less. The shape of the metal particles is not considered critical on the present invention. Any regular (e.g., spherical, polygonal, etc.) or irregular shapes are employable. Some shapes of the matrix particle will allow a higher packing density when targeted to a tissue, e.g. elongated particles will have in general a lower packing density than round spheres. Similarly, the particle size distribution of the metal particles in the matrix is not considered critical on the present invention although in some applications a certain size range may be of advantage. Different methods have been described for producing nanoparticles, including nucleation in solution (i.e. chemical synthesis) and vapor condensation or flame or spray techniques (Gutsch et al. (2002) KONA 20, 24-34; Axelbaum (2001) Powder Metall. 43(3), 323-325), but also more recently described techniques of laser ablation, vacuum evaporation on running liquids (VERL), and chemical vapor deposition (CVD) are suitable. Additionally or alternatively, an appropriate-sized nanoparticle distribution can be obtained by filtration or centrifugation. Any conventional method for grinding solids to the particle sizes useful in this invention can be employed.

An important characteristic of the matrix particles comprising a plurality of the metal nanoparticles of the present invention is their acoustical impedance, which renders them suitable for use as an ultrasound agent. Acoustic impedance (Z) is defined as the product of density (p) and speed of sound (c) in a medium (Kinsler et al., 1982, Fundamentals of acoustics. 3rd edition, John Wiley and sons, New York). The acoustical impedance of the metal nanoparticles of the present invention should be significantly higher than that of body tissues, the acoustical impedance of most body tissues being within the range of $1.3-1.7 \times 10^5$ g/cm$^2$s with a typical average of $1.58 \times 10^5$ g/cm$^2$s.

The present invention provides that the metal nanoparticles of the present invention have an acoustical impedance of at least $35 \times 10^5$ g/cm$^2$s, more particularly at least $50 \times 10^5$ g/cm$^2$s. The maximal acoustic impedance is envisaged to be around $100-120 \times 10^5$ g/cm$^2$s.

Examples of metals with an acoustical impedance within the above mentioned ranges, which are appropriate for incorporation in the matrix of the present invention are e.g. gold, silver, platinum, palladium, tungsten or tantalum, rhenium, or a mixture thereof, or alloys of metals, such as platinum and iridium alloys (see table 2 for a selected number of metals).

Table 2 Typical values for density (p), velocity (v) and acoustic impedance Z.

| | Z $10^6 kg \cdot m^2 \cdot s$ | $\rho$ $g/cm^3$ | $\nu$ $mm/\mu s$ |
|---|---|---|---|
| Platinum | 84.74 | 21.4 | 3.96 |
| Tungsten | 99.71 | 19.25 | 5.18 |
| Gold | 62.60 | 19.32 | 3.24 |
| Tantalum | 68.06 | 16.6 | 4.10 |
| Silver | 37.80 | 10.5 | 3.60 |

The metals for use in the metal nanoparticles are preferably metals which are chemically stable and non toxic or have been rendered chemically stable by an appropriate coating. Of particular interest in this regard are metals that combine the features of appropriate acoustical impedance with stability and non-toxity or limited toxicity. In other application wherein the metal particles are embedded or incorporated in the matrix, toxic metals with a high acoustic impedance can be used for in vivo applications. According to one embodiment the metal is a noble metal. According to a particular aspect of the invention, the metal is non-magnetic.

The matrix particles comprising a plurality of metal nanoparticles of the present invention can be used as an alternative for layers of individual targeted metal nanoparticles. It presents also an alternative for the protein-metal aggregates of WO02/11771. Herein, particles were associated by contacting assembled proteins with metal particles or metal salts. The aggregated particles being obtained have an irregular shape and size and lead to unequal distribution of the metal particles. As a consequence low densities of metal particles were obtained. The present invention allows the generation of matrix particles with defined size, shape and a controlled distribution and concentration in the matrix particle. Using the matrix particles of the present invention higher densities of particles can be achieved and consequently, superior reflection enhanced. The matrix particles of the present invention provide an improved and adjustable chemical and biological stability of the contrast agent. In addition, the matrix particles of the present invention are simple to produce and allow an increased process window. Furthermore, The matrix particles of the present invention can be efficiently modified with certain chemical or biological groups, e.g. hormone analogs, peptides mimicking ligands for receptors, which allows organ, tissue or cell specific targeting.

In order to cover a circular surface with a radius of 0,5 micrometer (3.14 square micrometer) with a monolayer of metal nanoparticles with a radius of 25 nm, about 360 nanoparticles with a radius of 25 nm are needed to cover said circular surface. [area circle / area nanoparticle = $\pi 500^2/\pi 25^2$ = 785398/1963 = 400, area circle/area square nanoparticle = $\pi 500^2/50^2$ = 785398/2500 = 314, hexagonal packing: (area circle $\cdot$ hexagonal packing density) / area nanoparticle = $(\pi 500^2 \cdot 0.9069) / \pi 25^2$ = (785398 $\cdot$0.9069) / 1963 = 362]. When using metal nanoparticles with a radius of 15 nm, 1000 particles would be needed to cover said circular surface. When, according to the present invention, the metal nanoparticles are clustered in a matrix particle with a radius of 0,5 micrometer, 360 metal nanoparticles (r=25 nm) of the above mentioned example occupy 5 % of the volume. The 1000 particles (r=15 nm) of the above mentioned example occupy 3 % of the volume.

By increasing the ratio of metal nanoparticles in a matrix particle, higher densities of metal nanoparticles per surface unit can be obtained as compared with the above mentioned monolayers. Thus, one embodiment of the invention related to matrix particles comprising at least 2%, at least 5%, at least 10%, at least 20 % or even at least 50 % (vol/vol) of metal nanoparticles.

"Matrix" in the context of the present invention, refers to any material in which a plurality of metal nanoparticles are able to reside and/or be restricted in movement. Matrices can be solid materials (rigid or flexible) but can also be liquids.

Examples of liquids are emulsions such as perfluorocarbon emulsions, as describes in US20040115192. The matrix particles can be homogeneous but also nonhomogeneous. Matrices can have an ordered structure, but this is not compulsory. Matrices can be porous, or hollow. Matrix materials with a certain density can be used depending on the desired rheological properties. Equally, matrices comprising gas bubbles can be envisaged in order to modulate the density of the matrix particle comprising the metal nanoparticles, especially when high concentrations of metals with a high density such as gold are present in the matrix particle.

Matrix particles comprising a plurality of metal nanoparticles refers to different arrangements wherein metal nanoparticles are distributed in a matrix or attached to a matrix. Examples hereof, are:

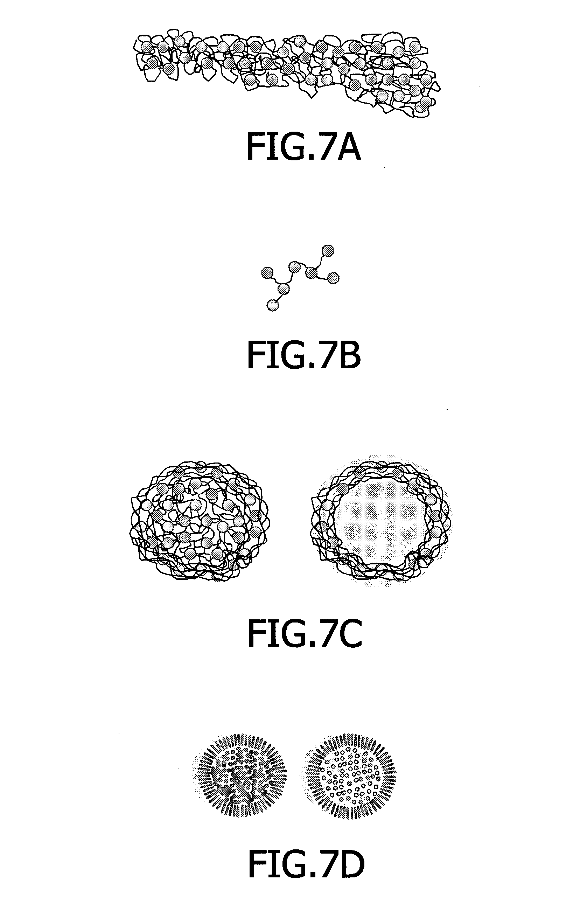

- Metal nanoparticles, which reside in the matrix. These can be covalently or non-covalently bound to the matrix, can be surrounded by the matrix constituents, or reside within pores in the matrix (figure 7a).
- Metal nanoparticles are arranged in each others' proximity by organic molecules used as an end capping layer on the metal particle (figure 7b).

- Metal nanoparticles are clustered in the shell of a micro bubble contrast agent or attached to the outside or inside of the shell of a micro bubble contrast agent (figure 7c).
- Metal nanoparticles are dispersed in droplets which are stabilized by a shell of polymer(s), lipid(s), surfactants or even proteins (figure 7d). These stabilized droplets can be used by themselves, e.g. when targeted, as an ultrasound contrast agent. By adding metal nanoparticles in, on or under the shell of the droplet an increase in reflection enhancement can be obtained. In this embodiment the matrix according to the present invention is the dispersion of the droplet. The droplets of the present invention have nanometer or eventually micrometer dimensions, have a low acoustic mismatch with body tissue. This is in contrast with prior art microbubbles having normally larger dimensions (micrometer) and a high acoustic mismatch with human tissue. Also the droplets of the present invention have an increased lifetime compared to prior art micobubbles.

According to the present invention, a plurality of metal particles are associated with a biocompatible and/or biodegradable matrix in order to cluster the metal nanoparticles. Matrices suitable for this end have been described in the art and include natural and synthetic carbohydrates, lipids, or physiologically tolerable synthetic polymers (including aptamers), surfactants, aqueous or organic liquids or mixtures or derivatives thereof.

Carbohydrates include natural and synthetic structural polysaccharides such as pectins and pectin fragments such as polygalacturonic acid, the glycosaminoglycans and heparinoids, e.g. heparin, heparan, keratan, dermatan, chondroitin and hyaluronic acid, dextrans, celluloses and the marine polysaccharides such as alginates, carrageenans and chitosans, and their derivatives.

Synthetic polymers that can be used as matrices include polyacrylates, polyvinylpyrollidone, polyamides, polyesters, polyethyleneglycols and polystyrenes. Moreover, matrices of multiblock copolymers are also envisaged, such as multiblocks of polylactic acid (PLA), polyglycolic acid (PGA), polyanhydrides, polyphosphazenes or polycaprolactone (PCL). According to a particular embodiment, the metal nanoparticles are first provided with a coating of one the above materials and subsequently further aggregated with a matrix of the same material or another material.

The matrix comprising the metal nanoparticles can also be of lipid nature. Lipid refers to a synthetic or naturally-occurring compound which is generally amphipathic and biocompatible. The lipids typically comprise a hydrophilic component and a hydrophobic component. Exemplary lipids include, for example, fatty acids, neutral fats, phosphatides, glycolipids, surface-active agents (surfactants), aliphatic alcohols, waxes, terpenes and steroids.

Lipids can arrange in micelles, being to colloidal entities formulated from lipids. In certain embodiments, the micelles comprise a monolayer or hexagonal H2 phase configuration. In other embodiments, the micelles may comprise a bilayer configuration. Lipids can also arrange in vesicles. These are spherical entity which is generally characterized by the presence of one or more walls or membranes which form one or more internal voids. An example of vesicles are those which comprise walls or membranes formulated from lipids. In these vesicles, the lipids may be in the form of a monolayer or bilayer, and the mono- or bilayer lipids may be used to form one or more mono- or bilayers. In the case of more than one mono- or bilayer, the mono- or bilayers may be concentric. Lipids may be used to form a unilamellar vesicle (comprised of one monolayer or bilayer), an oligolamellar vesicle (comprised of about two or about three monolayers or bilayers) or a multilamellar vesicle (comprised of more than about three monolayers or bilayers). Lipids can also arrange into liposomes, These are generally spherical clusters or aggregates of amphipathic compounds, lipid compounds, typically in the form of one or more concentric layers, for example, bilayers. They may also be referred to herein as lipid vesicles. The liposomes may be formulated, for example, from ionic lipids and/or non-ionic lipids. Liposomes which are formulated from non-ionic lipids may also be referred to as "niosomes."

In another embodiment the metal nanoparticles are incorporated in the wall of a vesicle which is of non-protein nature. Vesicles may be formulated, for example, from lipids, including the various lipids described before, or polymeric materials, including natural, synthetic and semi-synthetic polymers. Similarly, the vesicles prepared from polymers may comprise one or more concentric walls or membranes. The walls or membranes of vesicles prepared from polymers may be substantially solid (uniform), or they may be porous or semi-pourous. The vesicles described herein include such entities commonly referred to as, for example, liposomes, micelles, bubbles, microbubbles, microspheres, lipid-, or polymer coated bubbles, microbubbles and/or microspheres, microballoons, aerogels, clathrate bound vesicles. The internal void of the vesicles may be filled with a liquid (including, for example, an aqueous liquid), a gas, a gaseous precursor, and/or a solid or solute material, including, for example, a targeting ligand and/or a bioactive agent, as desired.

In another embodiment the metal nanoparticles are incorporated in the lumen of a vesicle. In this embodiment the vesicle functions merely as a shell around the matrix lumen comprising the nanoparticles, the composition of the material of the shell has no influence on the distribution of the metal nanoparticles and can be of any of the before mentioned constituent but can be also of protein nature.

In another embodiment the metal nanoparticles are localized on the outer or inner surface of the vesicle wall.

In another embodiment the metal nanoparticles are incorporated in both the wall of a vesicle and the lumen of a vesicle.

Thus depending on the localization of the metal nanoparticles, the lumen and/or the wall of the vesicle are the matrix particle according to the nomenclature of the present invention.

Matrix components may contain reactive functional groups such as amine, active ester, alcohol, thiol and carboxylate. Such functional groups may be used to attach onto the surface of the matrix particles biologically active molecules, especially bio-target specific agents. Suitable bio-target specific agents may be cell-, microorganism-, e.g. parasites such as nematodes or bacteria-, organ- or tissue specific molecules such as peptides or proteins, or antibodies or fragments thereof. Included within the term bio-target specific agents are molecules or functional groups directed at a specific foreign and/or toxic agent. The matrix may also comprise molecules affecting the charge, lipophilicity or hydrophilicity of the particle or its ability to enter through a cell membrane.

Depending on the envisaged application, the matrix component of the particles of the present invention is biodegradable in order to fragment the matrix into particles which can be secreted by the kidneys. In general the matrix component preferably remains intact for at least 30 minutes in order to allow the targeting to and imaging of an organ or diseased site. In particular embodiments, e.g. when using toxic metals, the matrix is heterogeneous, wherein the metal nanoparticles resides in a first material, e.g. coating, which is not biodegradable or degrades with a slow rate, said first material being associated with a second matrix material with a higher degradation rate. This allows the decomposition of the matrix particle to a size wherein the fragments of the first material are secreted by the kidney prior to the release of the metal nanoparticle from the first material.

A particular embodiment of the present invention relates to matrix particles, which are targeted to a particular organ or tissue. This can be achieved by attaching to the surface of the matrix particle a tissue or organ-specific molecule. One such molecule is an antibody, directed against an organ or tissue-specific antigen. For instance, such antibody can be a polyclonal or monoclonal antibody specific for a tumor-associated antigen or antimyosin. Non-limiting examples of polyclonal or monoclonal antibodies which can be used for conjugation include, especially, those that are principally directed at antigens found in the cell membrane. For example, suitable for the visualization of tumors are polyclonal or monoclonal antibodies per se, and/or their fragments (Fab, F(ab)$_2$), which are directed, for example, at the carcinoembryonal antigen (CEA), human choriogonadotrophin (.beta.-hCG) or other antigens found in tumors such as glycoproteins. Antimyosin, anti-insulin and antifibrin antibodies and/or fragments, inter alia, are also suitable. Alternatively, the molecule is a ligand for a receptor with a tissue-specific expression pattern. In the context of the present invention the term 'cellular marker' is used to refer to any molecule, which allows the identification of a specific cell, cell-type, tissue, type of tissue, organ or type of organ.

A further particular embodiment of the present invention relates to matrix particles, which are coated with a biologically or therapeutically active agent such as a drug or wherein the agent, e.g. drug, is encapsulated in the matrix, for use as drug-delivery agents or for combined diagnostic and therapeutic use Therapeutic agents can be selected over a wide range of drugs and are determined by the therapeutic target.

Optionally the matrix particles are further coated with a material that provides them with a hydrophilic coating to minimize the uptake of blood components and/or a steric barrier to particle-cell interaction, in order to minimize uptake by the liver. An example of such a material is the block copolymer known as tetronic 908 (US 4,904,497).

As described in US 6,165,440, ultrasonic waves can be used to obtain perforation of tumor blood vessels, microconvection in the interstitium, and/or perforation of cancer cell membrane. Following this principle, the matrix particles comprising the plurality of metal nanoparticles of the present invention can be used to obtain enhanced delivery of macromolecular therapeutic agents into cancer cells with minimal thermal and mechanical damage to normal tissues.

With the matrix particles of the present invention sufficient reflection enhancement can be obtained at the lower frequencies, which are normally used for ultrasonic imaging of organs or tissue deeper into the body. Using the matrix particles of the present invention, ultrasound imaging is performed using frequencies of about 22 MHz which allow reflection enhancements of 7 dB. A reflection enhancement of 7dB was found with a layer of clustered silver nanoparticles of 30 nm, which corresponds with a silver layer of 50 nm, as described in example 3. Thicker layers of clustered nanoparticles, thus also larger matrix particles, and metals which have a higher acoustic impedance will enhance the reflectivity even more.

Different combination of the matrix particles of the present invention can be envisaged such as populations of matrix particles comprising the same metal but differing in size of metal nanoparticles or differing in concentration of metal nanoparticle; matrices comprising a mixture of nanoparticles of different metals; mixtures of matrix particles of different composition and/or shape and other combinations thereof.

Matrix particles of this invention are optionally formulated into diagnostic compositions for enteral or parenteral administration. For example, parenteral formulations advantageously contain a sterile aqueous solution or suspension of coated metal particles according to this invention. Various techniques for preparing suitable pharmaceutical solutions and suspensions are known in the art. Such solutions also may contain pharmaceutically acceptable buffers and, optionally, additives such as, but not limited to electrolytes (such as sodium chloride) or antioxidants. Parenteral compositions may be injected directly or mixed with one or more adjuvants customary in galenicals, e.g. methyl cellulose, lactose, mannite, and/or surfactants, e.g., lecithins, Tween, Myrj. The matrix particles of the present invention can be used for a variety of imaging applications such as imaging, blood flow studies and blood analysis.

Conventional excipients are pharmaceutically acceptable organic or inorganic carrier substances suitable for parenteral, enteral or topical application, which do not deleteriously react with the agents. Suitable pharmaceutically acceptable adjuvants include but are not limited to water, salt solutions, alcohols, gum arabic, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxy-methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

Formulations for enteral administration may vary widely, as is well-known in the art. In general, such formulations include a diagnostically effective amount of the metal particles in aqueous solution or suspension. A syrup, elixir can be used wherein a sweetened vehicle is employed. Alternatively, the formulation can be in tablets, dragees, suppositories or capsules having talc and/or a carbohydrate carrier or binder, the carrier preferably being lactose and/or corn starch and/or potato starch.

For parenteral application, particularly suitable are injectable sterile solutions, preferably oil or aqueous solutions, as well as suspensions, emulsions, or implants, including suppositories. Ampoules are convenient unit dosages. The contrast agents containing the matrix particles comprising a plurality of metal nanoparticles are preferably used in parenteral application, e.g., as injectable solutions.

The diagnostic compositions of this invention are used in a conventional manner in ultrasound procedures. The diagnostic compositions are administered in a sufficient amount to provide adequate visualization, to a warm-blooded animal either systemically or locally to an organ or tissues to be imaged, then the animal is subjected to the medical diagnostic procedure. Such doses may vary widely, depending upon the diagnostic technique employed as well as the organ to be imaged. The contrast agents of this invention generally contain from 1 micromole to 1 mole, preferably 0.1 to 100 millimoles of metal per liter and are usually dosed in amounts of 0.001 to 100 micromoles, preferably 0.1 to 10 micromoles of metal per kilogram of body weight. They are administrable enterally and parenterally to mammals, including humans. Typically, diagnostic measurement is begun about 5-30 minutes after administration.

According to a specific embodiment of the present invention, the diagnostic composition of the invention are used for the imaging, i.e. the visualization of a tissue structure or target molecule in a tissue sample or organ ex vivo, i.e. on a tissue sample or organ that has been completely or partially isolated from the animal or human body.

The use of the contrast agents of the present invention are envisaged in a wide range of applications, including all applications which have been described for contrast imaging in the art, such as, visualization and diagnosis of tissues, parts thereof or structures therein, e.g. as tracers. For instance, contrast imaging is used in the visualization of the cardiovascular system, e.g. wall motion analysis, myocardial perfusion, identifying areas of infarction or ischemia in the myocardium, identifying blood clots, or the liver, e.g. liver function, detection of liver tumors. Other applications of contrast imaging envisaged include, but are not limited to visualization of the gastro-intestinal tract, visualization of tumors, identifying testicular and ovarian torsion, evaluation of renal and other transplanted organs, physiological pressure, and contrast agent-guided and controlled local drug delivery.

According to one aspect, the diagnostic compositions of the invention are used for combined use in different imaging methods. Thus, depending on their characteristics, the matrix particles comprising a plurality of metal nanoparticles of the invention may be appropriate for use in X-ray analysis. Thus, a particular embodiment of the invention relates to a diagnostic composition for use in combined imaging methods.

The following examples, may be understood in conjunction with the accompanying figures, incorporated herein by reference, in which:

Fig. 1 illustration of the parameters used in the theoretical model for reflection enhancement (of an incompressible layer).

Fig. 2 Theoretical calculated reflection enhancement of a 50 nm Au layer versus a 250 nm liquid-perfluorocarbon, lipid-encapsulated nanoparticulate emulsion layer (PFO) on top of a material with the same acoustic properties as average human tissue, as function of the frequency.

Fig. 3 Reflection enhancement of a 50 nm platinum layer, a 50 nm tungsten layer, a 50 nm gold layer and a 50 nm tantalum layer on top of a material with the same acoustic properties as average human tissue as a function of frequency.

Fig. 4 Dependency of layer thickness and frequency on the reflection enhancement of a gold contrast layer on top of a material with the same acoustic properties as average human tissue.

Fig. 5 Reflection enhancement of a liquid-perfluorocarbon layer versus gold and silver (on top of a material with the same acoustic properties as average human tissue) as function of its layer thickness.

Fig. 6 Integrated reflected intensity (peak area) of a 2 $\mu$m polymeric substrate and of a 2 $\mu$m polymeric substrate with clustered silver nanoparticles as a function of gain.

Fig. 7 Matrix particles comprising a plurality of metal nanoparticles.

Panel A: Metal nanoparticles embedded in the matrix particle.
Panel B: Metal nanoparticles bound to each other by organic molecules used as end capping layer on metal nanoparticles.
Panel C: Metal nanoparticles clustered in, on or under the shell of a micro bubble contrast agent [two bubbles are drawn, the second is a crossection].
Panel D: Metal nanoparticles dispersed in droplets.

The present invention is now further demonstrated by the following examples.

Examples

Example 1 - Reflection enhancement prediction as a function of the frequency of a 50 nm gold layer and a 250 nm PFO layer on top of a material with the same acoustic properties as average human tissue.
The reflection enhancement of a layer can be calculated using a mathematical model:

$$r(k) = r_{12} + \frac{t_{12} \cdot t_{21} \cdot r_{23} \cdot e^{2ikd}}{1 - r_{21} \cdot r_{23} \cdot e^{2ikd}} \qquad \text{[Equation 1]}$$

Wherein:

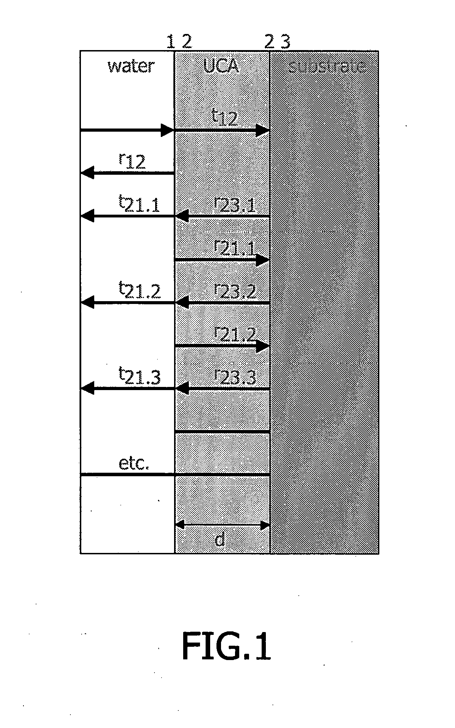

'r(k)' is the amplitude reflection coefficient of incompressible materials,
't' is the complex transmission coefficients between medium 1 (e.g. water), medium 2 (the ultrasound contrast layer/agent) and medium 3 (e.g. the substrate),
'r' is the complex reflection coefficients between medium 1 (e.g. water), medium 2 (the ultrasound contrast layer/agent) and medium 3 (e.g. the substrate) (see Figure 1),
'12' indicates the interface between medium 1 (e.g. water) and 2 (e.g. gold layer) and the direction of sound going from 1 to 2.
'k' is the wave number of the ultrasonic wave in the contrast layer.
'd' is the thickness of the contrast layer.

$$\text{And the enhancement is } 20.\log.(\,|r(k)|\,/\,|r_0|\,) \qquad \text{[Equation 2]}$$

wherein 'r(k)' is the amplitude reflection coefficient of incompressible materials, $r_0$ is the amplitude reflection coefficient of the substrate surface without the contrast agent.
Metal particles are not biodegradable and therefore these particles should be sufficiently small for excretion through the kidneys. 70 nm is considered to be the upper limit. PFO (perfluorocarbon) contrast agent droplets follow a different pathway, and the particles will dissolve and disappear through the lungs. In this examples a comparison is made between PFO particles at their actual size which is 250 nm and smaller gold particles of 50 nm.
The enhancement as calculated for a layer of perfluorocarbon emulsion droplets of 250 nm was in agreement with an ultrasound reflection enhancement observed for a layer of such particles on material with the acoustic properties of spleen tissue, e.g. $1.6 \times 10^5$ g/cm$^2$s, of which the acoustical impedance is very close to the average acoustical impedance of human tissue, e.g. $1.58 \times 10^5$ g/cm$^2$s.
Figure 2 shows the theoretical calculated reflection enhancement of a 50 nm Au layer versus a 250 nm liquid-perfluorocarbon, lipid-encapsulated nanoparticulate emulsion layer (PFO) on top of a blood clot, or another material with the same acoustic properties as average human tissue" as a function of the frequency. This graph indicates that the reflection enhancement of a 50 nm gold layer is still higher than the reflection enhancement of a 250 nm layer of a liquid-perfluorocarbon (PFO).

Example 2 - theoretical predicted reflection enhancement of a 50 nm platinum layer, a 50 nm tungsten layer, a 50 nm gold layer and a 50 nm tantalum layer on top of a material with the same acoustic properties as average human tissue as a function of the frequency.

Using the equations described above in example 1, the reflection enhancement of a 50 nm platinum layer, a 50 nm tungsten layer, a 50 nm gold layer and a 50 nm tantalum layer on top of blood clot, or another material with the same acoustic properties as average human tissueas a function of the frequency are calculated and shown in figure 3. Platinum, tungsten, and tantalum are, just like gold, good acoustic reflectors since have all a high density and a high longitudinal velocity, thus a high acoustic impedance difference with body tissue (table 3), obtaining a high reflection enhancement.

Table 3 Density, velocity and acoustic impedance values of platinum, tungsten, gold and tantalum

| | $\rho$ g/cm$^3$ | $\nu$ mm/$\mu$s | Z $10^6$kg·m$^2$·s |
|---|---|---|---|
| Platinum | 21.4 | 3.96 | 84.74 |
| Tungsten | 19.25 | 5.18 | 99.71 |
| Gold | 19.32 | 3.24 | 62.60 |
| Tantalum | 16.6 | 4.10 | 68.06 |

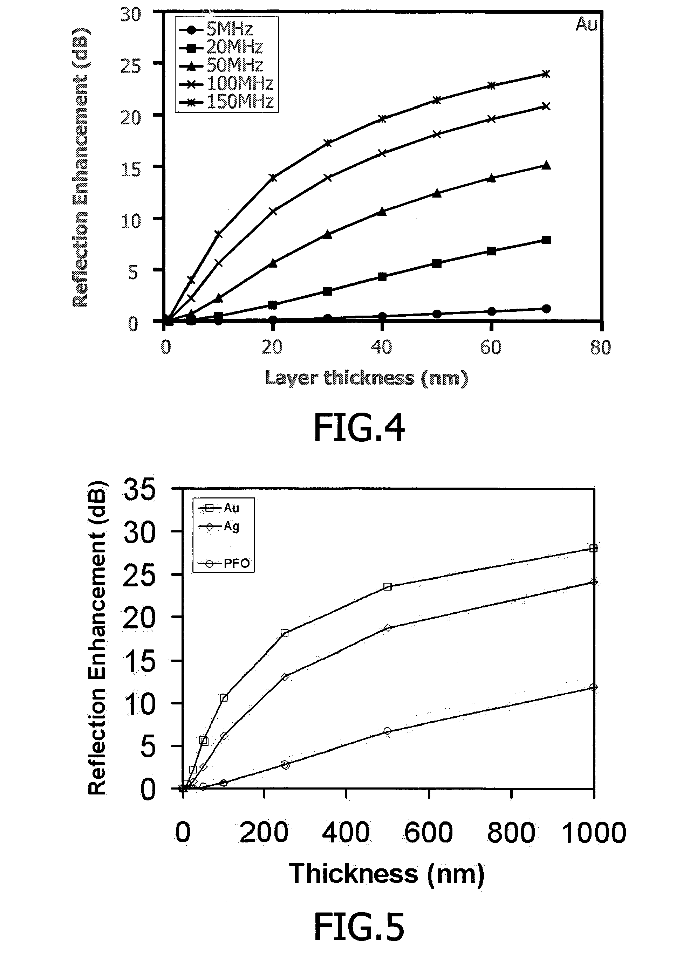

The dependence of the layer thickness and frequency on the reflection enhancement of a gold contrast layer on top of a blood clot, or another material with the same acoustic properties as average human tissueis shown in figure 4. These results indicate that increasing frequency (decreasing wavelength) results in an increase in reflection enhancement. Increasing the metal layer thickness results in an increase in reflection enhancement. Since the penetration of ultrasound is dependent upon the frequency of the transducer, high frequencies (obtaining a higher reflection enhancement) cannot be used for medical ultrasound imaging of organs and other tissues deeper inside the body.

Another way to increase the reflection enhancement can be obtained by a significant increase of the diameter of the metal particles. In figure 5 the reflection enhancement of a liquid-perfluorocarbon layer versus gold and silver is shown as a function of its layer thickness. PFO, state-of-the-art contrast agents, e.g. for the absorbed layer approach, based on shell- encapsulated droplets of perfluorocarbons, is used as comparison for metal nanoparticle contrast agents. These calculations indicate that the reflection enhancement of metals with a high acoustic impedance, such as silver and gold, have a much higher reflection enhancement than the PFO contrast agent at the same layer thickness.

However, it is not acceptable to use significant larger metal particles, e.g. 250 nm metal particles, since such larger particles will not be excreted through the filter of the kidneys and will accumulate into the body.

Example 3 - Measurement of the reflection enhancement of clustered silver nanoparticles on a polymeric substrate

The present example illustrates that the clustering of small metal nanoparticles provides an acceptable alternative for metal particles with a diameter above 70 nm.

Clustered silver nanoparticles were deposit on a polymeric substrate of 2 micrometer. The amount of silver is measured with an X-ray photoelectron spectrometer (XPS) with a spot size of 5 mm and is shown in table 4.

Table 4 Density, velocity and acoustic impedance values of platinum, tungsten, gold and tantalum.

| measurement | $\mu$g Ag/cm$^2$ | at. Ag $10^{17}$/cm$^2$ |
|---|---|---|
| 1 | 50.5 | 2.82 |
| 2 | 50.9 | 2.84 |

The amount of silver of the deposited silver nanoparticles of 30 nm corresponds with a silver layer of 50 nm.

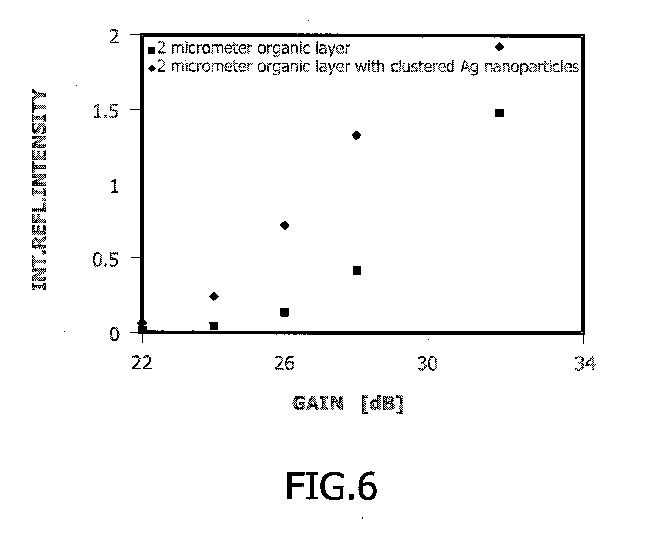

A Digital Ultrasound Imaging System of Taberna Pro Medicum equipped with a 22 MHz transducer was used to measure the reflection of the polymeric substrate with and without the clustered silver nanoparticles of 30 nm. The integrated reflected intensity (peak area) of a 2 $\mu$m polymeric substrate and of a 2 $\mu$m polymeric substrate with the clustered silver nanoparticles as a function of the gain is shown in Figure 6. The clustered silver nanoparticles of 30 nm enhances the reflectivity of the polymeric substrate with 7 dB.

**Claims**

1. A contrast agent for medical diagnostics and imaging comprising a plurality of metal nanoparticles, wherein said plurality of metal nanoparticles are encapsulated in a non-proteinaceous biocompatible or biodegradable matrix particle and/or attached to a non-proteinaceous biocompatible or biodegradable matrix particle, the matrix of the matrix particles being selected from the group consisting of a carbohydrate, a lipid, a synthetic polymer, an aqueous liquid, a surfactant and an organic liquid, or a mixture thereof, wherein said metal nanoparticles have a diameter between 1 and 100 nm **characterised in that** said metal nanoparticles are present in a concentration of at least 2% volume/volume in said matrix.

2. The contrast agent comprising a plurality of metal nanoparticles according to claim 1, wherein said matrix is the shell of a vesicle.

3. The contrast agent according to claim 1 or 2, wherein said metal nanoparticles have an acoustic impedance of at least 35.105 g/cm2s.

4. The contrast agent according to any of claims 1 to 3, wherein said metal nanoparticles have an acoustic impedance of above 50.105 g/cm2s.

5. The contrast agent according to claims 1 to 4, wherein said metal nanoparticles have a diameter between 1 and 50 nm.

6. The contrast agent according to any of claims 1 to 5 wherein said metal is non-magnetic.

7. The contrast agent according to claim 6 wherein said non-magnetic metal is selected from the group consisting of gold, silver, platinum, palladium, tungsten or tantalum, rhenium, or a mixture thereof.

8. The contrast agent according to any of claims 1 to 7 wherein said metal is a noble metal.

9. The contrast agent according to any of claims 1 to 8 wherein said matrix particles have a diameter between 1 and 8 micrometer.

10. The contrast agent according to any of claims 1 to 8 wherein said matrix particles have a diameter between 25 and 250 nanometer.

11. The contrast agent according to any of claims 1 to 10, wherein one or more targeting molecules are attached to said matrix particle.

12. The contrast agent according to any of claims 1 to 10, wherein one or more targeting molecules are attached to the surface of the metal nanoparticles.

13. The use of a non-proteinaceous matrix particles comprising a plurality of metal nanoparticles, wherein said metal nanoparticles have a diameter between 1 and 100 nm and wherein said metal nanoparticles are present in a concentration of at least 2% volume/volume in said matrix, for the manufacture of an ultrasound contrast agent, wherein said matrix is being selected from the group consisting of a carbohydrate, a lipid, a synthetic polymer, an aqueous liquid and an organic liquid, or a mixture thereof.

**Patentansprüche**

1. Kontrastmittel zur medizinischen Diagnose und Bildgebung mit einer Vielzahl von Metall-Nanopartikeln, wobei die genannte Vielzahl von Metall-Nanopartikeln in einem nicht-proteinhaltigen biokompatiblen oder biologisch abbaubarem Matrixpartikel verkapselt sind und/oder an einem nicht-proteinhaltigen biokompatiblen oder biologisch abbaubarem Matrixpartikel angebracht sind, wobei die Matrix der Matrixpartikel ausgewählt wird aus der Gruppe bestehend aus einem Kohlehydrat, einem Lipid, einem synthetischen Polymer, einer wässrigen Flüssigkeit, einem Tensid und einer organischen Flüssigkeit oder einer Mischung hiervon, wobei die genannten Metall-Nanopartikel einen Durchmesser zwischen 1 und 100 nm haben, **dadurch gekennzeichnet, dass** die genannten Metall-Nanopartikel in einer Konzentration von mindestens 2 Volumenprozent in der genannten Matrix vorhanden sind.

2. Kontrastmittel mit einer Vielzahl von Metall-Nanopartikeln nach Anspruch 1, wobei die genannte Matrix die Hülle eines Vesikels ist.

3. Kontrastmittel nach Anspruch 1 oder 2, wobei die genannten Metall-Nanopartikel eine akustische Impedanz von mindestens 35,105 g/cm2s haben.

**4.** Kontrastmittel nach einem der Ansprüche 1 bis 3, wobei die genannten Metall-Nanopartikel eine akustische Impedanz über 50,105 g/cm2s haben.

**5.** Kontrastmittel nach Anspruch 1 bis 4, wobei die genannten Metall-Nanopartikel einen Durchmesser zwischen 1 und 50 nm haben.

**6.** Kontrastmittel nach einem der Ansprüche 1 bis 5, wobei das genannte Metall nicht-magnetisch ist.

**7.** Kontrastmittel nach Anspruch 6, wobei das nicht-magnetische Metall ausgewählt wird aus der Gruppe bestehend aus Gold, Silber, Platin, Palladium, Wolfram oder Tantal, Rhenium, oder einer Mischung hiervon.

**8.** Kontrastmittel nach einem der Ansprüche 1 bis 7, wobei das genannte Metall ein Edelmetall ist.

**9.** Kontrastmittel nach einem der Ansprüche 1 bis 8, wobei die genannten Matrixpartikel einen Durchmesser zwischen 1 und 8 Mikrometer haben.

**10.** Kontrastmittel nach einem der Ansprüche 1 bis 8, wobei die genannten Matrixpartikel einen Durchmesser zwischen 25 und 250 Nanometer haben.

**11.** Kontrastmittel nach einem der Ansprüche 1 bis 10, wobei ein oder mehrere Zielmoleküle an dem genannten Matrixpartikel angebracht ist.

**12.** Kontrastmittel nach einem der Ansprüche 1 bis 10, wobei ein oder mehrere Zielmoleküle an der Oberfläche der Metall-Nanopartikel angebracht sind.

**13.** Verwendung von nicht-proteinhaltigen Matrixpartikeln mit einer Vielzahl von Metall-Nanopartikeln, wobei die genannten Metall-Nanopartikel einen Durchmesser zwischen 1 und 100 nm haben und wobei die genannten Metall-Nanopartikel in einer Konzentration von mindestens 2 Volumenprozent in der genannten Matrix vorhanden sind, für die Herstellung eines Ultraschall-Kontrastmittels, wobei die genannte Matrix ausgewählt wird aus der Gruppe bestehend aus einem Kohlenhydrat, einem Lipid, einem synthetischen Polymer, einer wässrigen Flüssigkeit und einer organischen Flüssigkeit oder einer Mischung hiervon.


**Revendications**

**1.** Agent de contraste pour imagerie et diagnostics médicaux comprenant une pluralité de nanoparticules métalliques, dans lequel ladite pluralité de nanoparticules métalliques sont encapsulées dans une particule de matrice biocompatible ou biodégradable non protéinique et/ou attachées à une particule de matrice biocompatible ou biodégradable non protéinique, la matrice de particules de matrice étant sélectionnée dans le groupe se composant d'un glucide, un lipide, un polymère synthétique, un liquide aqueux, un surfactant et un liquide organique, ou un mélange de ceux-ci, dans lequel lesdites nanoparticules métalliques ont un diamètre entre 1 et 100 nm, **caractérisé en ce que** lesdites nanoparticules métalliques sont présentes dans une concentration d'au moins 2% volume/volume dans ladite matrice.

**2.** Agent de contraste comprenant une pluralité de nanoparticules métalliques selon la revendication 1, dans lequel ladite matrice est la coque d'une vésicule.

**3.** Agent de contraste selon la revendication 1 ou 2, dans lequel lesdites nanoparticules métalliques ont une impédance acoustique d'au moins 35,105 g/cm$^2$s.

**4.** Agent de contraste selon l'une quelconque des revendications 1 à 3, dans lequel lesdites nanoparticules métalliques ont une impédance acoustique supérieure à 50,105 g/cm$^2$s.

**5.** Agent de contraste selon l'une quelconque des revendications 1 à 4, dans lequel lesdites nanoparticules métalliques ont un diamètre entre 1 et 50 nm.

**6.** Agent de contraste selon l'une quelconque des revendications 1 à 5, dans lequel ledit métal est non magnétique.

7.  Agent de contraste selon la revendication 6, dans lequel ledit métal non magnétique est sélectionné dans le groupe se composant d'or, argent, platine, palladium, tungstène ou tantale, rhénium ou un mélange de ceux-ci.

8.  Agent de contraste selon l'une quelconque des revendications 1 à 7, dans lequel ledit métal est un métal noble.

9.  Agent de contraste selon l'une quelconque des revendications 1 à 8, dans lequel lesdites particules de matrice ont un diamètre entre 1 et 8 micromètres.

10. Agent de contraste selon l'une quelconque des revendications 1 à 8, dans lequel lesdites particules de matrice ont un diamètre entre 25 et 250 nanomètres.

11. Agent de contraste selon l'une quelconque des revendications 1 à 10, dans lequel une ou plusieurs molécules de ciblage sont attachées à ladite particule de matrice.

12. Agent de contraste selon l'une quelconque des revendications 1 à 10, dans lequel une ou plusieurs molécules de ciblage sont attachées à la surface des nanoparticules métalliques.

13. Utilisation d'une particule de matrice non protéinique comprenant une pluralité de nanoparticules métalliques, dans laquelle lesdites nanoparticules métalliques ont un diamètre entre 1 et 100 nm et dans laquelle lesdites nanoparticules métalliques sont présentes dans une concentration d'au moins 2% volume/volume dans ladite matrice, pour la fabrication d'un agent de contraste ultrasonore, dans laquelle ladite matrice est sélectionnée dans le groupe se composant d'un glucide, un lipide, un polymère synthétique, un liquide aqueux et un liquide organique, ou un mélange de ceux-ci.

# FIG.1

FIG.2

FIG.3

FIG.4

FIG.5

FIG.6

FIG.7A

FIG.7B

FIG.7C

FIG.7D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020136693 A
- US 20030082237 A
- WO 0211771 A
- WO 2004068405 A
- US 20030003054 A
- US 20040115192 A
- US 4904497 A
- US 6165440 A

**Non-patent literature cited in the description**

- **HALL C.S. et al.** *J. Acoust. Soc. Am.,* 2000, vol. 108 (6), 3049-3057
- **BEKEREDJIAN et al.** *Ultrasound Med. & Biol.,* 2002, vol. 28 (5), 691-695
- **GUTSCH et al.** *KONA,* 2002, vol. 20, 24-34
- **AXELBAUM.** *Powder Metall.,* 2001, vol. 43 (3), 323-325
- **KINSLER et al.** Fundamentals of acoustics. John Wiley and sons, 1982